# EUROPÄISCHE PATENTSCHRIFT

(11) **EP 1 633 609 B1**
(45) Veröffentlichungstag und Bekanntmachung des Hinweises auf die Patenterteilung: **20.02.2008**
(21) Anmeldenummer: 04729413.7
(22) Anmeldetag: 26.04.2004
(51) Int. Cl.: B60R 22/12, B60R 11/02, A61B 5/024, A61B 5/18

(54) **SENSORANORDNUNG ZUR ANBRINGUNG AN EINEM GURT, INSBESONDERE AN EINEM SICHERHEITSGURT EINES KRAFTFAHRZEUGS**
SENSOR ARRANGEMENT FOR APPLYING TO A BELT, ESPECIALLY TO A SAFETY BELT OF A MOTOR VEHICLE
DISPOSITIF DETECTEUR A FIXER SUR UNE CEINTURE, NOTAMMENT UNE CEINTURE DE SECURITE D'UN VEHICULE AUTOMOBILE

(30) Priorität: 18.06.2003 DE 10327753
(43) Veröffentlichungstag der Anmeldung: 15.03.2006
(73) Patentinhaber: paragon AG, 33129 Delbrück (DE)
(72) Erfinder: RODEMER, Klaus, 36369 Lautertal (DE)
(74) Vertreter: Lelgemann, Karl-Heinz
(86) Internationale Anmeldenummer: PCT/EP2004/004378
(87) Internationale Veröffentlichungsnummer: WO 2004/110829

(56) Entgegenhaltungen:
- EP-A- 0 712 604
- DE-A- 10 209 695
- US-A- 5 060 977
- PATENT ABSTRACTS OF JAPAN Bd. 0081, Nr. 40 (M-305), 29. Juni 1984 (1984-06-29) -& JP 59 038153 A (NIPPON DENSO KK), 1. März 1984 (1984-03-01)

## Beschreibung

Die Erfindung bezieht sich auf eine Sensoranordnung zur Anbringung an einem Sicherheitsgurt eines Kraftfahrzeugs nach dem Oberbegriff des Anspruchs 1.

Bei aus dem Stand der Technik bekannten Sensoranordnungen sind die Sensoren derselben am Sicherheitsgurt angebracht bzw. befestigt, indem beispielsweise den Sicherheitsgurt durchdringende Halterungen od.dgl. vorgesehen sind; allen bekannten Halterungen bzw. Fixierungen der Sensoren am Sicherheitsgurt ist gemeinsam, dass einerseits die mechanischen Eigenschaften des Gurtes beeinträchtigt werden, wobei des weiteren die Trageeigenschaften des Sicherheitsgurtes insoweit negativ verändert werden, als durch auf der Unterseite des Sicherheitsgurts vorstehende Elemente der Halterungen bzw. Fixierungen die glatte Struktur der dem Fahrzeuginsassen zugewandten Unterseite des Gurtes gestört ist. Diese glatte Struktur der Unterseite des Gurtes ist jedoch von wesentlicher Bedeutung, um das notwendige leichtgängige Gleiten des Sicherheitsgurtes über die Umlenkvorrichtungen der Sicherheitsgurtanordnungen und den Körper des Kraftfahrzeuginsassen zu gewährleisten. Die Beeinträchtigung dieser Gleiteigenschaften des Sicherheitsgurtes kann zur Folge haben, dass der Sicherheitsgurt nach Bewegungen des Fahrzeuginsassen von den Retraktoren der Sicherheitsgurtanordnung nicht mehr optimal an den Körper des Kraftfahrzeuginsassen herangezogen wird. Bei einem etwaigen Unfall od.dgl. würde der Sicherheitsgurt damit seine Schutzfunktion - wenn überhaupt - nur ungenügend entfalten.

Eine Sensoranordnung nach dem Oberbegriff des Anspruchs 1 ist aus der JP-A-59038153 bekannt. Diese Sensoranordnung besteht aus einem einzigen Sensor, der mittels einer lösbaren Befestigung an unterschiedlichen Stellen in einem Bereich auf der nach außen gerichteten Oberseite des Sicherheitsgurts angebracht werden kann. Hierzu ist die Oberseite des Sicherheitsgurts in dem genannten Bereich mit einer Art Klett- oder Haftverschlussband versehen. Auf der Rückseite des Sensors ist ein entsprechendes Gegenstück angebracht, welches mit dem Klett- oder Haftverschlussband in Eingriff bringbar ist. Der Sensor kann, in Abhängigkeit davon, wer den Sicherheitsgurt benutzt, an einer geeigneten Position innerhalb des vom Klett- oder Haftverschlussband begrenzten Bereichs fixiert werden.

Die EP-A-0 712 604 beschreibt eine Sensoranordnung, die mehrere einzelne Sensoren aufweisen kann, wobei für die Auswertung derjenige einzelne Sensor ausgewählt wird, der über das aussagekräftigste der unterschiedlichen Sensorsignale verfügt. Irgendwelche Anregungen hinsichtlich der konstruktiven Ausgestaltung der einzelnen Sensoren oder der Art ihrer Befestigung an einem Sicherheitsgurt gehen aus dieser Druckschrift nicht hervor.

Aus der US-A-5 060 977 ist es bekannt, einen Magneten, der für den Betrieb eines in einem Sicherheitsgurtsystem vorgesehenen Sensors benötigt wird, an einem Sicherheitsgurt zu befestigen. Hierzu ist eine Magnethalterung vorgesehen, die an einer Seite des Sicherheitsgurts angenäht wird. In welcher Weise die Nähte in bezug auf den Sicherheitsgurt verlaufen und auf welcher Seite des Sicherheitsgurts die Anbringung des Magneten erfolgt, geht aus dieser Druckschrift nicht hervor.

Die DE-A-102 09 695 zeigt einen Sensor, der an einem Sicherheitsgurt angebracht ist, wobei die gezeigten Anbringungsarten jeweils mit Veränderungen auf derjenigen Seite des Sicherheitsgurts einhergehen, mit der der Sicherheitsgurt am Fahrzeuginsassen bzw. an dessen Bekleidung anliegt.

Der Erfindung liegt die Aufgabe zugrunde, eine Sensoranordnung zur Anbringung an einem Sicherheitsgurt eines Kraftfahrzeugs zu schaffen, die zu keiner bzw. zu einer geringeren Beeinträchtigung der für die Schutzfunktion des Sicherheitsgurtes wesentlichen Gleiteigenschaften an der Unterseite desselben führt.

Diese Aufgabe wird erfindungsgemäß von einer Sensoranordnung mit den Merkmalen des Anspruchs 1 gelöst.

Das Gehäuse lässt sich vorteilhaft aus einem Kunststoff ausgestalten.

Um zu verhindern, dass der Sicherheitsgurt in denjenigen Bereichen, an denen die Sensoren der Sensoranordnung vorgesehen sind, partiell geschwächt wird, ist es zweckmäßig, in diesen erreichen den Sicherheitsgurt durch Einweben zusätzlicher Fäden zu verstärken.

Alternativ kann eine Gurtverstärkung in den die Sensoren der Sensoranordnung aufweisenden Bereichen des Sicherheitsgurts erreicht werden, indem in diesen Bereichen Fäden mit besseren mechanischen Eigenschaften, z.B. einer erhöhten Bruchlast und/oder Elastizität, für die Ausgestaltung des Sicherheitsgurts eingesetzt werden.

Die Sensoren der Sensoranordnung können z.B. als Mikrofone ausgebildet sein. Dann ist es möglich, ohne störende Handgriffe vornehmen zu müssen, die ggf. die Kontrolle des Fahrzeugs beeinträchtigen, bequem zu telefonieren.

Alternativ oder zusätzlich können als Herzfrequenz-Körpertemperaturmesser od.dgl. ausgebildete Sensoren vorgesehen sein. So kann beispielsweise bei einem Signal eines Herzfrequenzmessers, welches die Fahruntüchtigkeit des Kraftfahrzeuglenkers anzeigt, so in den Betrieb des Kraftfahrzeugs eingegriffen werden, dass möglichst keine bzw. geringe Schäden entstehen.

Die Sensoren der Sensoranordnung sind zweckmäßigerweise an in den Sicherheitsgurt integrierte Leiter angeschlossen, in die bzw. aus denen die erforderliche Betriebsenergie der Sensoranordnung und Signale der Sensoren induktiv ein- bzw. auskoppelbar ist bzw. sind. Die Sensoren der Sensoranordnung können hierbei in verschiedenen Topologien angeschlossen sein. Dies hängt von der Bauart der Sensoren ab. Sensoren, die einen ausreichenden Signalwert liefern, werden einzeln oder mit einem gemeinsamen Bezugsleiter kontaktiert. Sensoren mit niedrigem Signalwert benötigen häufig eine separate Spannungszuführung. Integrierte Sensoren können zu einem gemeinsamen Bus zusammen geschaltet werden.

Die Betriebsenergie und die Signale können vorteilhaft im Bereich eines von zwei Retraktoren oder eines Gurtschlosses der Sicherheitsgurtanordnung ein- bzw. auskoppelbar sein.

Die in den Sicherheitsgurt eingewebten Leiter haben hinsichtlich ihres Bruch- und ihres Dehnverhaltens besonders vorteilhafte Eigenschaften, wenn die Leiter auf einen originalen Webfaden des Sicherheitsgurts aufgesponnen werden. Die Steigung und der Abstand des Leiters beim Spinnen bestimmen dann das Dehnverhalten. Des weiteren sind die Leiter bei dieser vorteilhaften Ausführungsform der Erfindung von außen nicht sichtbar.

Die Verbindung zwischen den Sensoren einerseits und den in den Sicherheitsgurt integrierten Leitern andererseits kann vorteilhaft mittels Flexleitern oder Leitgummis realisiert werden.

Um den für den jeweiligen Zweck am besten positionierten Sensor der Sensoranordnung zu ermitteln, ist es vorteilhaft, wenn eine Auswahleinrichtung vorgesehen ist, mittels der der für die Signalqualität am besten positionierte Sensor der Sensoranordnung ermittelt werden kann, wobei dann das Signal dieses Sensors zur Weiterleitung auswählbar ist.

Zur weiteren Verbesserung des Ausgangssignals sollte die Auswahleinrichtung die zwei für die Signalqualität am besten positionierten Sensoren der Sensoranordnung, z.B. den ersten oberhalb und den ersten unterhalb der Signalquelle angeordneten Sensor auswählen und aus den Signalen dieser beiden Sensoren das Ausgangssignal der Sensoranordnung zusammenfassen. Die Signale der Sensoren können aufbereitet werden, wobei hierzu eine Verstärkung und Normierung der Signale, aber auch eine Linearisierung und eine Filterung der Signale gehören kann. Mit Hilfe der Auswahleinrichtung kann durch eine Signalbewertung der am besten platzierte Sensor bzw. die am besten platzierte Sensorgruppe ohne Berücksichtigung der Gurtlänge oder der Sitzposition ausgewählt werden.

Das Ausgangssignal des ausgewählten Sensors bzw. der ausgewählten Sensorgruppe kann unter Einbeziehung der Signale der weiteren Sensoren der Sensoranordnung, vorzugsweise nach beliebigen mathematischen Verfahren, bearbeitet werden, wobei hierdurch beispielsweise die Möglichkeit besteht, Windgeräusche, z.B. in einem Cabrio, aus einem Mikrofonsignal herauszufiltern.

Die Auswahleinrichtung kann unter Berücksichtigung der Ausgangssignale eines Sitzpositionsfühlers und/oder eines Fahrzeuginsassengewichtsfühlers und/oder eines Sicherheitsgurtauszuglängenfühlers arbeiten und in Abhängigkeit hiervon die Auswahl des bzw. der am besten positionierten Sensoren treffen.

Im folgenden wird die Erfindung anhand einer Ausführungsform unter Bezugnahme auf die Zeichnung näher erläutert.

Es zeigen:
- Figur 1: eine Prinzipdarstellung eines Sicherheitsgurtes, der mit einer erfindungsgemäßen Sensoranordnung ausgerüstet ist; und
- Figur 2: den Schnitt A - A in Figur 1.

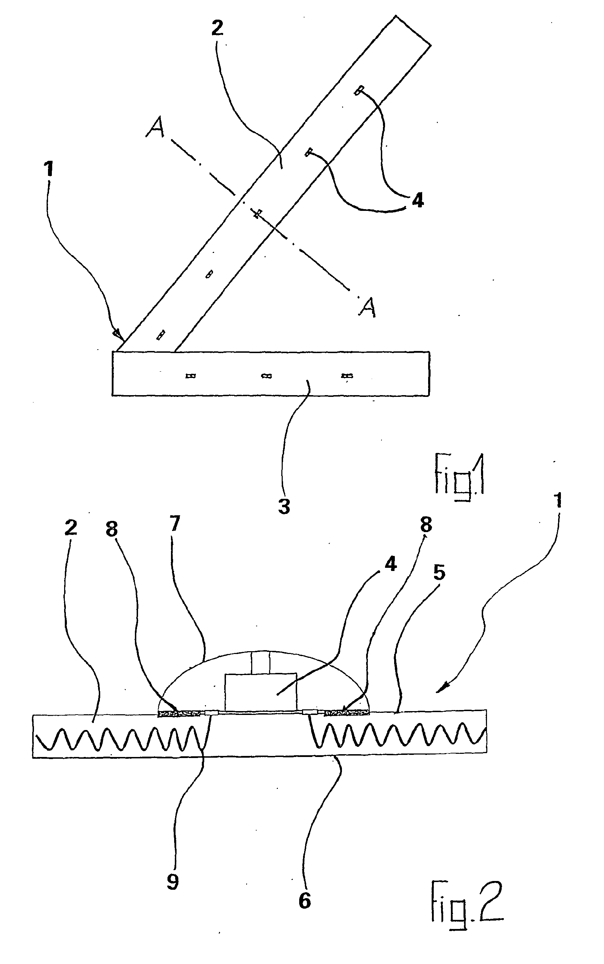

Ein in Figur 1 in Prinzipdarstellung gezeigter Sicherheitsgurt 1 gliedert sich in ein schräg aufwärts verlaufendes Schultergurtteil 2 und ein etwa waagerecht verlaufendes Bauchgurtteil 3.

Auf der in Figur 1 sichtbaren, dem Körper einer durch den Sicherheitsgurt 1 geschützten Person abgewandten Vorderseite des Sicherheitsgurts 1 ist eine Sensoranordnung vorgesehen, zu der eine Vielzahl von Sensoren 4 gehört. Im dargestellten Ausführungsbeispiel sind die Sensoren 4 der Sensoranordnung mit etwa dem gleichen Abstand in Längsrichtung sowohl des Schultergurtteils 2 als auch des Bauchgurtteils 3 angeordnet. Wie bereits erwähnt, sitzen die Sensoren 4 auf der dem Kraftfahrzeuginsassen abgewandten Oberseite 5 des Sicherheitsgurts 1.

Die Unterseite 6 des Sicherheitsgurts 1 ist durch die Sensoren 4 der Sensoranordnung nicht beeinträchtigt und völlig ungestört. Der Lauf des Sicherheitsgurts 1 in Umlenkeinrichtungen ud.dgl. wird somit die Sensoren 4 der Sensoranordnung nicht beeinträchtigt.

Im dargestellten Ausführungsbeispiel weist jeder Sensor 4 der Sensoranordnung ein Gehäuse 7 auf, in welchem er aufgenommen ist und mittels dem er auf der Oberseite 5 des Sicherheitsgurts 1 gehaltert ist. Je nach Ausgestaltung des Sensors 4 ist es auch möglich, diesen nicht mittels eines Gehäuses 7 sondern mittels einer andersartigen Halterung an der Oberseite 5 des Sicherheitsgurts 1 anzubringen.

Wie sich am besten aus Figur 2 ergibt, ist das den Sensor 4 aufnehmende Gehäuse 7 mittels einer Verbindung 8 mit dem die Oberseite 5 des Sicherheitsgurts 1 ausbildenden Werkstoff fest verbunden. Bei der Verbindung 8 kann es sich um eine Schweißverbindung, um eine Klebeverbindung und auch um eine Naht od.dgl. handeln.

Der Sicherheitsgurt 1 kann an denjenigen Stellen, an denen die Sensoren 4 der Sensoranordnung vorgesehen sind, mittels eingewebter Fäden verstärkt werden, um etwaige, aufgrund der Anbringung der Sensoren 4 auftretende Schwächungen des Sicherheitsgurts 1 auszugleichen.

Bei den in den Figuren 1 bzw. 2 dargestellten Sensoren 4 kann es sich beispielsweise um Mikrofone handeln, mittels denen es möglich ist, dass der Fahrzeuginsasse, ohne dass er irgendwelche störenden und seine Konzentration auf die Steuerung des Fahrzeugs beeinträchtigende Handgriffe vornehmen müsste, telefoniert. Selbstverständlich kann die aus den Sensoren 4 gebildete Sensoranordnung an jedem Sicherheitsgurt innerhalb des Kraftfahrzeugs vorgesehen sein.

Alternativ oder zusätzlich können als Herzfrequenz-, Körpertemperatursensoren od.dgl. ausgebildete Sensoren 4 vorgesehen sein.

Zur Energieversorgung und zum Signalanschluss der Sensoren 4 sind bei dem in den Figuren dargestellten Ausführungsbeispiel in den Sicherheitsgurt 1 Leiter 9 integriert. Mittels dieser Leiter 9 kann die erforderliche Betriebsenergie den Sensoren 4 zugeführt werden, wobei mittels der Leiter 9 auch die Signalausgabe der Sensoren 4 stattfinden kann.

Sowohl die Einkoppelung der für den Betrieb der Sensoren 4 erforderlichen Betriebsenergie als auch die Auskoppelung der Signale der Sensoren 4 kann induktiv vorgenommen werden, wobei als Einkoppel- bzw. Auskoppelstelle einer von zwei Retraktoren des Sicherheitsgurts 1 dienen kann.

Die in den Sicherheitsgurt 1 integrierten Leiter 9 können um einen Webfaden des Sicherheitsgurts 1 gesponnen sein.

Der Anschluss zwischen den Leitern 9 einerseits und den Sensoren 4 andererseits kann mittels Flexleitern, Leitgummis ud.dgl. realisiert sein.

Zu der durch die Sensoren 4 gebildeten Sensoranordnung gehört eine in den Figuren 1 und 2 nicht gezeigte Auswahleinrichtung, die Signale von allen Sensoren 4 der Sensoranordnung erhält. In der in den Figuren nicht gezeigten Auswahleinrichtung kann festgestellt werden, wie die Signalqualität der einzelnen Sensoren 4 ist. Es ist möglich, mittels der Auswahleinrichtung denjenigen oder die zwei Sensoren 4 auszuwählen, dessen bzw. deren Signalqualität am besten ist. Sofern die beiden Sensoren 4 mit der besten Signalqualität ausgewählt werden, können deren Signale zu einem Ausgangssignal zusammengefasst werden.

Darüber hinaus ist es möglich, dieses Ausgangssignal weiter zu verfeinern, indem die Signale der nicht für die Zusammensetzung des Ausgangssignal ausgewählten Sensoren 4 berücksichtigt und zur Bearbeitung des Ausgangssignals herangezogen werden.

An die Auswahlvorrichtung kann ein Sitzpositionsfühler und ein Fahrzeuginsassengewichtsfühler angeschlossen werden. Deren Ausgangssignale können bei der Auswahl des bzw. der für die Signalqualität am besten positionierten Sensoren 4 der Sensoranordnung eingesetzt werden, wobei für diese Auswahl auch das Ausgangssignal eines Sicherheitsgurtauszuglängenfühlers berücksichtigt werden kann.

## Patentansprüche

1. Sensoranordnung zur Anbringung an einem Sicherheitsgurt (1) eines Kraftfahrzeugs, die an der dem Körper des Kraftfahrzeuginsassen abgewandten Oberseite (5) des Sicherheitsgurts (1) angebracht ist, wobei die dem Körper des Kraftfahrzeuginsassen zugewandte Unterseite des Sicherheitsgurts (1) frei und unverändert bleibt, **dadurch gekennzeichnet, daß** die Sensoranordnung mehrere Sensoren (4) aufweist, die an der dem Körper des Kraftfahrzeuginsassen abgewandten Oberseite des Sicherheitsgurts (1) angebracht sind, und daß jeder Sensor(4)in einem Gehäuse (7) aufgenommen ist, das mit dem die Oberseite (5) des Sicherheitsgurts (1) ausbildenden Werkstoff verschweißbar oder verklebbar oder das an die Oberseite (5) des Sicherheitsgurts (1) annähbar ist.

2. Sensoranordnung nach Anspruch 1, bei der das Gehäuse (7) jedes Sensors (4) aus einem Kunststoff ausgebildet ist.

3. Sensoranordnung nach Anspruch 1 oder 2, bei der der Sicherheitsgurt (1) im Bereich zumindest eines, vorzugsweise jedes, Sensors (4) der Sensoranordnung mit einer Gurtverstärkung versehen ist.

4. Sensoranordnung nach Anspruch 3, deren Gurtverstärkung bzw. Gurtverstärkungen in Form zusätzlich in den Sicherheitsgurt (1) eingewebter Fäden ausgebildet ist bzw. sind.

5. Sensoranordnung nach Anspruch 3, deren Gurtverstärkung bzw. Gurtverstärkungen **dadurch** ausgebildet ist bzw. sind, dass in deren Bereich bzw. Bereichen Fäden mit besseren mechanischen Eigenschaften, z.B. einer erhöhten Bruchlast und/oder Elastizität, eingesetzt sind.

6. Sensoranordnung nach einem der Ansprüche 1 bis 5, deren Sensoren (4) Mikrofone sind.

7. Sensoranordnung nach einem der Ansprüche 1 bis 6, zu deren Sensoren (4) Herzfrequenz-, Körpertemperaturmesser und/oder dergleichen gehören.

8. Sensoranordnung nach einem der Ansprüche 1 bis 7, deren Sensoren (4) an in den Sicherheitsgurt (1) integrierte Leiter (9) angeschlossen sind, in die bzw. aus denen die erforderliche Betriebsenergie der Sensoranordnung und Signale der Sensoren (4) induktiv ein- bzw. auskoppelbar ist bzw. sind.

9. Sensoranordnung nach Anspruch 8, bei der die Betriebsenergie und die Signale im Bereich eines von zwei Retraktoren oder eines Gurtschlosses der Sicherheitsgurtanordnung ein- bzw. auskoppelbar ist bzw. sind.

10. Sensoranordnung nach Anspruch 8 oder 9, bei der die in den Sicherheitsgurt (1) integrierten Leiter um einen Webfaden des Sicherheitsgurts (1) gesponnen sind.

11. Sensoranordnung nach einem der Ansprüche 8 bis 10, deren Sensoren (4) mittels Flexleitern mit den in den Sicherheitsgurt (1) integrierten Leitern (9) verbunden sind.

12. Sensoranordnung nach Anspruch 11, deren Sensoren (4) mittels Leitgummis mit den in den Sicherheitsgurt (1) integrierten Leitern (9) verbunden sind.

13. Sensoranordnung einem der Ansprüche 1 bis 12, bei der eine Auswahleinrichtung vorgesehen ist, mittels der der für die Signalqualität am besten positionierte Sensor (4) der Sensoranordnung ermittelbar und das Signal dieses Sensors (4) zur Weiterleitung auswählbar ist.

14. Sensoranordnung nach Anspruch 13, bei der mittels der Auswahleinrichtung die zwei für die Signalqualität am besten positionierten Sensoren (4) der Sensoranordnung, z.B. der erste oberhalb und der erste unterhalb der Signalquelle angeordnete Sensor (4), ermittelbar und die Signale dieser beiden Sensoren (4) zu einem Ausgangssignal zusammenfassbar sind.

15. Sensoranordnung nach Anspruch 13 oder 14, bei der das Ausgangssignal des ausgewählten Sensors (4) der Sensoranordnung bzw. das aus den Signalen zweier oder mehrerer ausgewählter Sensoren (4) zusammengefasste Ausgangssignal der Sensoranordnung unter Einbeziehung der Signale der weiteren Sensoren (4) der Sensoranordnung, vorzugsweise nach beliebigen mathematischen Verfahren, bearbeitbar ist.

16. Sensoranordnung nach einem der Ansprüche 13 bis 15, deren Auswahleinrichtung an einen Sitzpositionsfühler und/oder einen Fahrzeuginsassengewichtsfühler angeschlossen ist, wobei die Ausgangssignale dieses bzw. dieser Fühler bei der Auswahl des bzw. der am besten positionierten Sensoren (4) der Sensoranordnung, vorzugsweise gemeinsam mit einem Ausgangssignal eines Sicherheitsgurtauszuglängenfühlers, berücksichtigbar ist bzw. sind.

## Claims

1. A sensor arrangement for application to a safety belt (1) of a motor vehicle, which is attached to the upper side (5) of the safety belt facing away from the body of the motor vehicle passenger, wherein the lower side of the safety belt (1) facing the body of the motor vehicle passenger remains free and unchanged, **characterised in that** the sensor arrangement comprises a plurality of sensors (4) which are attached to the upper side of the safety belt (1) facing away from the motor vehicle passenger and that each sensor (4) is held in a housing (7) which can be welded or adhesively bonded to the material forming the upper side (5) of the safety belt (1) or which can be sewn onto the upper side (5) of the safety belt (1).

2. The sensor arrangement according to claim 1, wherein the housing (7) of each sensor (4) is formed from a plastic.

3. The sensor arrangement according to claim 1 or 2, wherein the safety belt (1) is provided with a belt reinforcement in the area of at least one, preferably each sensor (4) of the sensor arrangement.

4. The sensor arrangement according to claim 3, wherein the belt reinforcement or belt reinforcements is or are configured in the form of threads additionally woven into the safety belt (1).

5. The sensor arrangement according to claim 3, wherein the belt reinforcement or belt reinforcements is or are configured by inserting threads having better mechanical properties, e.g. an elevated breaking load and/or elasticity in the region or regions thereof.

6. The sensor arrangement according to any one of claims 1 to 5, wherein the sensors (4) are microphones.

7. The sensor arrangement according to any one of claims 1 to 6, wherein the sensors (4) include heart rate, body temperature meters and/or the like.

8. The sensor arrangement according to any one of claims 1 to 7, wherein the sensors (4) are connected to conductors (9) integrated in the safety belt (1) into which or from which the required operating energy of the sensor arrangement and signals from the sensors (4) can be inductively coupled in or out.

9. The sensor arrangement according to claim 8, wherein the operating energy and the signals can be coupled in or out in the area of one of two retractors or a belt lock of the safety belt arrangement.

10. The sensor arrangement according to claim 8 or 9, wherein the conductors integrated in the safety belt (1) are spun around a warp thread of the safety belt (1).

11. The sensor arrangement according to any one of claims 8 to 10, wherein the sensors (4) are connected to the conductors (9) integrated in the safety belt (1) by means of flexiconductors.

12. The sensor arrangement according to claim 11, wherein the sensors (4) are connected to the conductors (9) integrated in the safety belt (1) by means of rubber conductors.

13. The sensor arrangement according to any one of claims 1 to 12, wherein a selection device is provided by which means the sensor (4) of the sensor arrangement which is best positioned for the signal quality can be determined and the signal from this sensor (4) can be selected for further transmission.

14. The sensor arrangement according to claim 13, wherein the two sensors (4) of the sensor arrangement which are best positioned for the signal quality, e.g. the first sensor (4) arranged above and the first sensor arranged below the signal source, can be determined and the signals from these two sensor (4) can be combined to form an output signal.

15. The sensor arrangement according to claim 13 or 14, wherein the output signal of the selected sensor (4) of the sensor arrangement or the output signal of the sensor arrangement combined from the signals or two or more selected sensors (4) can be processed using the signals of further sensors (4) of the sensor arrangement, preferably according to any mathematical method.

16. The sensor arrangement according to any one of claims 13 to 15, wherein the selection device is connected to a seat position sensor and/or a vehicle passenger weight sensor, wherein the output signals of this or these sensors should be taken into account when selecting the best positioned sensor(s) (4) in the sensor arrangement, preferably jointly with an output signal of a safety belt extension length sensor.

## Revendications

1. Ensemble de capteurs destiné à être monté sur une ceinture de sécurité (1) d'un véhicule automobile, qui est monté sur la face supérieure (5) de la ceinture de sécurité (1) qui est opposée au corps du passager du véhicule automobile, la face inférieure de la ceinture de sécurité (1) dirigée vers le corps du passager du véhicule restant libre et inchangée, **caractérisé en ce que** l'ensemble de capteurs comporte plusieurs capteurs (4), qui sont montés sur la face supérieure de la ceinture de sécurité (1) qui est opposée au corps du passager et **en ce que** chaque capteur (4) est logé dans un boîtier (7) susceptible d'être soudé ou collé avec la matière formant la face supérieure (5) de la ceinture de sécurité (1) ou susceptible d'être cousu sur la face supérieure (5) de la ceinture de sécurité (1).

2. Ensemble de capteurs selon la revendication 1, sur lequel le boîtier (7) de chaque capteur (4) est conçu dans une matière plastique.

3. Ensemble de capteurs selon la revendication 1 ou 2, sur lequel, dans la région d'au moins un, de préférence de chaque capteur (4) de l'ensemble de capteurs, la ceinture de sécurité (1) est munie d'un renfort de ceinture.

4. Ensemble de capteurs selon la revendication 3, dont le renfort de ceinture ou les renforts de ceinture est ou sont conçu(s) sous la forme de fils tissés en supplément dans la ceinture de sécurité (1).

5. Ensemble de capteurs selon la revendication 3, dont le renfort de ceinture ou les renforts de ceinture est ou sont concu(s) en ce que dans leur région ou leurs régions, sont utilisés des fils présentant de meilleures propriétés mécaniques, par exemple une charge de rupture et/ou une élasticité renforcées.

6. Ensemble de capteurs selon l'une quelconque des revendications 1 à 5 dont les capteurs (4) sont des microphones.

7. Ensemble de capteurs selon l'une quelconque des revendications 1 à 6, dont les capteurs (4) incluent des systèmes de mesure de la fréquence cardiaque ou de la température corporelle.

8. Ensemble de capteurs selon l'une quelconque des revendications 1 à 7, dont les capteurs (4) sont raccordés à des conducteurs (9) intégrés dans la ceinture de sécurité (1), dans lesquels ou à partir desquels l'énergie de fonctionnement nécessaire de l'ensemble de capteurs et des signaux des capteurs (4) sont susceptibles d'être injectés, respectivement extraits.

9. Ensemble de capteurs selon la revendication 8, sur lequel l'énergie de fonctionnement et les signaux sont susceptibles d'être injectés, respectivement extraits dans la région d'un rétracteur ou d'un verrouillage de ceinture du dispositif de ceinture de sécurité.

10. Ensemble de capteurs selon la revendication 8 ou 9, sur lequel les conducteurs intégrés dans la ceinture de sécurité (1) sont filés autour d'un fil tissé de la ceinture de sécurité (1).

11. Ensemble de capteurs selon l'une quelconque des revendications 8 à 10, dont les capteurs (4) sont reliés aux conducteurs (9) intégrés dans la ceinture de sécurité (1) au moyen de conducteurs souples.

12. Ensemble de capteurs selon la revendication 11, dont les capteurs (4) sont reliés au conducteurs (9) intégrés dans la ceinture de sécurité (1) au moyen de caoutchoucs conducteurs.

13. Ensemble de capteurs selon l'une quelconque des revendications 1 à 12, sur lequel il est prévu un dispositif de sélection, au moyen duquel le capteur (4) de l'ensemble de capteurs qui est le mieux positionné pour la qualité du signal peut être recherché et le signal dudit capteur (4) peut être sélectionné pour sa retransmission.

14. Ensemble de capteurs selon la revendication 13, sur lequel au moyen du dispositif de sélection, les deux capteurs (4) de l'ensemble de capteurs qui sont le mieux positionnés pour la qualité du signal, par exemple le premier capteur (4) situé au-dessus et le premier situé en dessous de la source de signaux peuvent être recherchés et les signaux desdits capteurs (4) peuvent être regroupés en un signal de sortie.

15. Ensemble de capteurs selon la revendication 13 ou 14, sur lequel le signal de sortie du capteur (4) sélectionné de l'ensemble de capteurs ou le signal de sortie de l'ensemble de capteurs, regroupé à partir des signaux de deux ou de plusieurs capteurs (4) sélectionnés est susceptible d'être traité sous implication des signaux provenant des autres capteurs (4) de l'ensemble de capteurs, de préférence selon des méthodes mathématiques quelconques.

16. Ensemble de capteurs selon l'une quelconque des revendications 13 à 15, dont le dispositif de sélection est raccordé sur un palpeur de la position assise et/ou sur un palpeur du poids du passager du véhicule, lors de la sélection du ou des capteur (s) (4) le mieux positionné de l'ensemble de capteurs, les signaux de sortie dudit ou desdits palpeurs étant susceptible(s) d'être pris en considération en commun avec un signal de sortie d'un palpeur de la longueur d'extraction de la ceinture de sécurité.
